# EUROPEAN PATENT SPECIFICATION

(11) **EP 2 326 374 B1**
(45) Date of publication and mention of the grant of the patent: **04.03.2020**
(21) Application number: 09765629.2
(22) Date of filing: 19.06.2009
(51) Int. Cl.: A61M 15/00, B05B 11/00, A61M 11/06

(54) **INHALER**
INHALATOR
INHALATEUR

(30) Priority: 20.06.2008 EP 08011228
(43) Date of publication of application: 01.06.2011
(73) Proprietor: Boehringer Ingelheim International GmbH, 55216 Ingelheim (DE)
(72) Inventor: SPALLEK, Michael, 55216 Ingelheim Am Rhein (DE); WACHTEL, Herbert, 55216 Ingelheim am Rhein (DE); BICKMANN, Deborah, 55430 Urbar (DE)
(74) Representative: Simon, Elke Anna Maria
(86) International application number: PCT/EP2009/004435
(87) International publication number: WO 2009/153049

(56) References cited:
- WO-A-00/33902
- WO-A-01/78818
- WO-A-2004/091704
- WO-A-2005/014175
- WO-A-2005/079997
- WO-A-2007/141203
- WO-A1-95/20414
- WO-A1-2007/141201
- DE-U1- 29 920 790

## Description

The present invention relates to an inhaler with an add-on device having a chamber for the intermediate storage of an atomised medicament preparation.

The present invention relates in particular to a so-called soft mist inhaler (SMI), i.e. an inhaler of the type which produces only a relatively slowly spreading spray mist (aerosol). Inhalers of this kind for the purposes of the present invention are in particular inhalers in which an aerosol is delivered at a speed of less than 2 m/s, preferably less than 1.5 m/s and most preferably less than 1 m/s (in each case measured at a distance of 10 cm from a dispensing nozzle).

The starting point of the present invention is an inhaler as described in principle in WO 91/14468 A1, WO 2005/014175 A1 and specifically in WO 97/12687 A1 (Figs. 6a, 6b). The known inhaler comprises as a reservoir for a medicament preparation which is to be atomised an insertible, rigid container having an inner bag containing the medicament preparation and a pressure producing means having a drive spring for conveying and nebulising the medicament preparation. Atomisation is carried out without the use of a propellant gas, namely by the force of the drive spring. This inhaler is an SMI in the sense of the present invention.

A problem with inhalers and SMIs in general is that the triggering of the atomisation of the medicament preparation and breathing in have to be coordinated. This may be difficult for the individual user. In particular, it has been found that such coordination is very difficult specifically for children. Studies have shown that the aerosol produced by unskilled users or, for example, children is often not optimally inhaled.

WO 2005/079997 A1 discloses a propellant-free atomizer comprising a spring-powered pressure generator for conveying and nebulizing a fluid from a reservoir into an aerosol with a low cloud velocity so that the user can inhale the aerosol very slowly. Upon inhalation air is drawn through an air opening in the mouthpiece of the atomizer. The atomizer comprises a valve device so that the air opening can be closed for a reversed air flow, so that the user instantly realizes if he mistakenly exhales into the mouthpiece.

WO 2004/091704 A1 discloses an add-on device for the intermediate storage of an atomised medicament preparation in a chamber. The add-on device is used in a so-called metered dose inhaler (MDI). The MDI comprises a pressurised container which contains the medicament preparation that is to be atomised and propellant gas. On actuation, the medicament preparation is expelled by means of the propellant gas at comparatively high pressure and correspondingly high speed and with a high mass flow. As a result, the expulsion is very brief, lasting in particular for less than 0.4 s, usually for about 0.15 to 0.36 s. The short expulsion time is disadvantageous for inhalation as the process of breathing in for inhalation usually lasts considerably longer. The comparatively high speed of more than 2 m/s often even above 8 m/s, at which the aerosol is usually delivered by an MDI, is also disadvantageous for receiving it into the lungs as the particles (droplets) of the aerosol are largely deposited on the walls of the user's throat as a result of the high speed during direct inhalation.

The known add-on device is provided for an MDI and serves to slow down the aerosol, particularly by lengthening the flow path. For this reason, add-on devices of this kind are also known as spacers. Moreover, the add-on device serves for intermediate storage of the aerosol produced so that the user has sufficient time to inhale the aerosol.

Other spacers for use with MDIs are disclosed in WO 95/20414 A1 and WO 00/33902 A1.

WO 01/78818 A2 discloses a nasal inhaler comprising a chamber, a container with a dispensing portion disposed in a dosage inlet passage into the chamber, wherein the chamber additionally comprises a one way air inlet valve and an outlet with an air exit passage.

WO 2007/141201 A1 discloses an adapter for the attachment of a portable inhaler in a breathing circuit of a ventilator system.

WO 2007/141203 A1 discloses a portable inhaler which can be used for nasal administration by fitting a nosepiece onto the mouthpiece of the inhaler.

Object of the present invention is to provide an inhaler, most preferably an SMI, with an add-on device comprising a chamber for intermediate storage of an atomised medicament preparation, thereby making it possible to simplify inhalation even of aerosols delivered at low speed and/or preventing or at least minimising problems occurring in the coordination of breathing in and the operation of an inhaler.

The above object is achieved by means of an inhaler according to claim 1. Advantageous embodiments are subject of the subclaims.

The present invention is based on the idea of combining a portable nebuliser for propellant-free nebulising of a medicament preparation or an SMI with an add-on device comprising a chamber for intermediate storage of the aerosol produced, the chamber being arranged downstream of a delivery nozzle of the inhaler.

It has been found that thanks to the add-on device, even in an inhaler which produces the aerosol that is to be inhaled over a comparatively long time, preferably more than 1 second, and/or at comparatively low speed, preferably less than 2 m/s, most preferably less than 1.5 m/s (measured at a distance of 10 cm from a delivery nozzle), it is possible to achieve surprisingly improved inhalation of the active substance, particularly in small children or other people who have problems of coordination. Coordinating the actuation of the inhaler, i.e. the production of the aerosol, and breathing in is made substantially easier. The aerosol is produced by the inhaler and sprays into the chamber of the add-on device. The user can then inhale the aerosol by breathing in as deeply as possible but without any compulsion of coordination or synchronisation.

The proposed solution allows better defined inhalation of the active substance with a content of active substance which is, in the last analysis, higher on average and/or fluctuates less, this active substance being deposited in the lungs. This allows improved therapy of children and/or a broadening of the indication or the use of other medicament preparations. Thus, in the last analysis, it renders a propellant free inhaler or SMI universally usable.

Preferably, the add-on device has a valve so as to prevent the user from breathing out into the inhaler or chamber, i.e. to prevent air from flowing back from the delivery side of the add-on device into the chamber.

Further advantages, features, properties and aspects of the present invention will become apparent from the claims and the following description of a preferred embodiment according to the drawings, wherein:
- Fig. 1: is a schematic section through an inhaler in the untensioned state;
- Fig. 2: is a schematic section through the inhaler, rotated through 90° relative to Fig. 1, in the tensioned state;
- Fig. 3: is a schematic section through the inhaler, corresponding to Fig. 1, with the add-on device attached;
- Fig. 4: is a schematic exploded view of the nebuliser and the add-on device with different accessories; and
- Fig. 5: is a schematic view of test results.

In the figures, the same reference numerals have been used for identical or similar parts where corresponding or comparable properties and advantages are achieved, even if the relevant description has not been repeated.

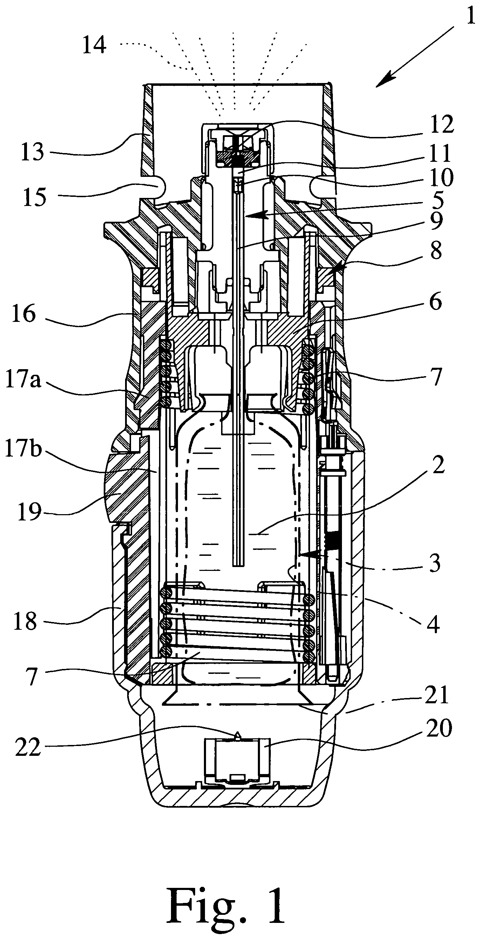

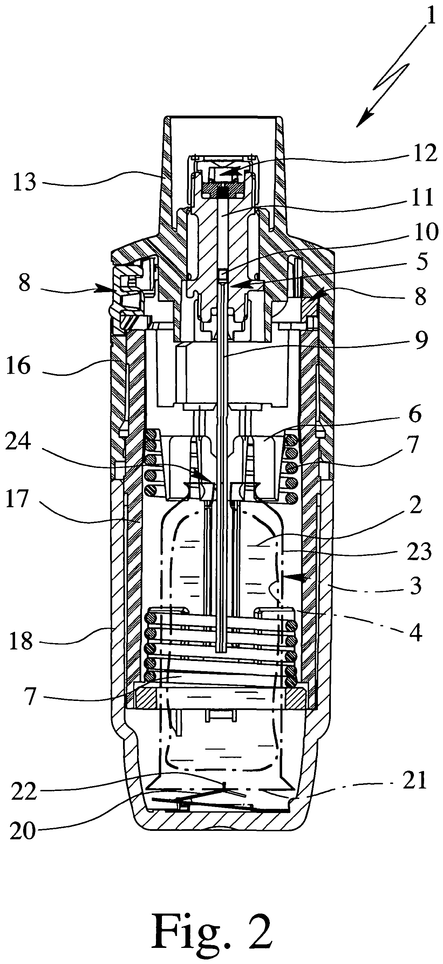

Figs. 1 and 2 show a proposed portable inhaler 1 for the propellant free nebulisation of a medicament preparation 2 in a schematic view in the untensioned state (Fig. 1) and in the tensioned state (Fig. 2). Figs. 1 and 2 show the inhaler 1 with a container 3 holding the medicament preparation 2.

During the nebulisation of the medicament preparation 2, preferably a liquid, a respirable aerosol 14 (Fig. 1) is formed which can be breathed in or inhaled by a user or patient (not shown). Normally, inhalation takes place at least once a day, but particularly several times a day, preferably at specified intervals of time, more particularly depending on the complaint suffered by the patient.

The inhaler 1 comprises the preferably insertible and optionally exchangeable container 3 holding the medicament preparation 2. The container 3 thus forms a reservoir for the medicament preparation 2 which is to be nebulised. Preferably, the container 3 contains a sufficient quantity of medicament preparation 2 or active substance for several doses of the medicament preparation 2, i.e. to allow a number of nebulisations or applications. A typical container 3 as disclosed in WO 96/06011 A1 holds a volume of about 2 to 10 ml. The preferred construction of the container 3 is disclosed in WO 00/49988 A2.

The container 3 is preferably substantially cylindrical or cartridge-shaped and can be inserted into the inhaler 1 from below, after it has been opened, and optionally exchanged. It is preferably of rigid construction, the medicament preparation 2 being contained in particular in a collapsible bag 4 in the container 3.

The inhaler 1 also comprises a pressure generator 5, for conveying and nebulising the medicament preparation 2, particularly in a predetermined and optionally adjustable dosage amount in each case.

The inhaler 1 or pressure generator 5 comprises in particular a holder 6 for the container 3 and associated drive spring 7 which is only partly shown, preferably having an associated locking element 8 which is manually operable to release it, a conveying element, preferably a conveying tube 9 in the form of a capillary, with an optional valve, particularly a non-return valve 10, a pressure chamber 11 and/or a delivery nozzle 12, particularly in the region of a mouthpiece 13.

The container 3 is fixed in the inhaler 1 by means of the holder 6, particularly by a clamping or latching action, such that the conveying tube 9 protrudes into the container 3. The holder 6 may be constructed such that the container 3 can be exchanged.

When the drive spring 7 is axially tensioned, the holder 6 with the container 3 and the conveying tube 9 is moved downwards in the figures and the medicament preparation 2 - or more precisely the next dose - is sucked out of the container 3 into the pressure chamber 11 of the pressure generator 5 through the non-return valve 10.

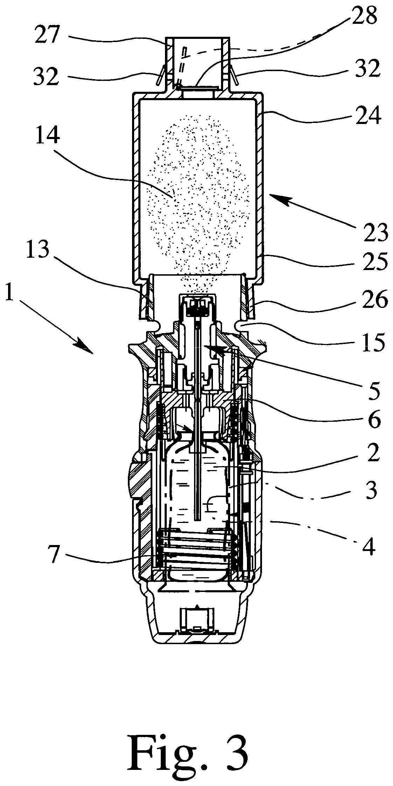

During the subsequent release of tension after actuation of the locking element 8, the medicament preparation 2 in the pressure chamber 11 is placed under pressure by moving the conveying tube 9 back up, with the non-return valve 10 now closed, by releasing the tension on the drive spring 7, so that this conveying tube 9 now acts as a pressure ram. This pressure expels the medicament preparation 2 through the delivery nozzle 12, where it is nebulised into the preferably respirable aerosol 14, as shown in Figs. 1 and 3.

The user or patient (not shown) can inhale the aerosol 14, while supply air can be sucked into the mouthpiece 13 through at least one supply air opening 15.

During the nebulisation process the container 3 is moved back into its original position by the drive spring 7. The container 3 thus performs a lifting movement during the tensioning process and during the nebulisation process.

The inhaler 1 comprises in particular a first housing part (upper part) 16 and an inner part 17 which is rotatable relative thereto (Fig. 2) having an upper part 17a and a lower part 17b (Fig. 1), while a second housing part (lower part) 18, which is in particular manually operable or rotatable, is releasably attached, in particular pushed onto the inner part 17, preferably by means of a safety closure or retaining element 19. In particular, the safety closure or retaining element 19 is constructed such that accidental opening of the inhaler 1 or removal of the second housing part 18 is prevented. In particular, in order to release the second housing part 18, the retaining element 19 has to be pressed in against spring force. In order to insert and/or replace the container 3, the second housing part 18 can be detached from the inhaler 1. The second housing part 18 preferably forms a cap-like lower housing part and/or engages around or over a lower free end portion of the container 3.

The second housing part 18 can be rotated relative to the first housing part 16, whereby the inner part 17 is also rotated. In this way the drive spring 7 is tensioned in the axial direction by means of a gear (not shown in detail) acting on the holder 6. During tensioning the container 3 is moved axially downwards or with its end portion (further) into the second housing part 18 or towards the end face thereof, until the container 3 assumes an end position shown in Fig. 2. In this state the drive spring 7 or inhaler 1 is clamped and locked.

The inhaler 1 preferably has a device for forcibly ventilating the container 3.

When tensioning first takes place, the container 3 is preferably pierced in its base or opened. In particular, an axially acting spring 20 arranged in the housing part 18 comes to abut on the container base 21 and with a piercing element 22 pierces the container 3 or an in particular gas tight seal provided in the base for ventilation purposes when contact is first made.

The device for forcible ventilation is thus formed in this case by the piercing element 22, which is held or formed by the spring 20. However, other design solutions are also possible.

It should be noted that during the piercing for ventilation purposes only the outer shell of the container 3 is opened. The bag 4 containing the medicament preparation 2 remains undamaged. As the medicament formulation 2 is removed from the bag 4 through the conveying tube 9 the flexible bag 4 collapses. For pressure equalisation, ambient air can flow into the container 3 through the ventilation or piercing opening.

In order to use the inhaler 1, first of all the container 3 has to be inserted. This is preferably done by removing or pulling out the second housing part 18. The container 3 is then axially inserted or pushed into the inner part 17. At the same time the container 3 is opened at the head end or attached. This is done by means of the conveying element, i.e. the conveying tube 9, which pierces a seal preferably provided at the head end of the container 3 and is then inserted through a septum at the head end of the container 3 into the interior of the bag 4. Thus the fluidic connection between the container 3, or more accurately between the bag 4 in the container 3, via the conveying tube 9 to the pressure generator 5 or pressure chamber 11 is produced.

Then the second housing part 18 is pushed on again. The inhaler 1 can now be tensioned for the first time. At this stage the container 3 is then pierced at its base by the piercing element 22, i.e. forcibly ventilated, as explained previously.

Before being used for the first time and after the container 3 has been inserted and fluidically connected, the inhaler 1 is preferably tensioned and actuated several times. This so-called priming displaces any air present in the medicament preparation 2 in the conveying tube 9 and in the pressure generator 5 to the delivery nozzle 12. The inhaler 1 is then ready for inhalation.

The quantity of medicament preparation 2 delivered per spray or nebulisation process is preferably about 10 µl to 50 µl, more particularly about 10 µl to 20 µl, most preferably about 15 µl.

The drive spring 7 is preferably installed in a biased state in order to achieve a high spring pressure. In the proposed inhaler 1 the pressurisation and conveying of the medicament preparation 2 during the nebulisation process namely takes place preferably only by spring force, and more particularly only by the force of the drive spring 7.

The inhaler 1 is preferably constructed such that the medicament preparation 2 in the pressure generator 5 or in the pressure chamber 11 reaches a pressure of 5 MPa to 60 MPa, particularly about 10 MPa to 50 MPa during delivery. Particularly preferably, during the delivery or nebulisation of the medicament preparation 2, a pressure of about 5 MPa to 60 MPa, more particularly about 10 to 30 MPa, is reached at the delivery nozzle 12 or at the nozzle openings thereof. The medicament preparation 2 is then converted into the aerosol 14, the droplets of which have an aerodynamic diameter of up to 20 µm, preferably about 3 µm to 10 µm. The nebulising activity or nebulising effect is achieved or further assisted by preferably intercepting jets delivered by the delivery nozzle 12.

The inhaler 1 is preferably constructed such that the aerosol 14 is delivered at low speed, particularly at a speed of less than 2 m/s, most preferably about 1.6 m/s or less (in each case measured at a distance of 10 cm from the delivery nozzle 12). The inhaler 1 is thus preferably in the form of an SMI. The low delivery speed can be obtained or assisted by intercepting jets of the medicament preparation 2, which are delivered by the delivery nozzle 12 and/or by a suitable choice of spring force.

Particularly preferably, the construction of the inhaler 1 is such that the aerosol generation lasts for more than 0.7 s, more preferably at least about 1 s and in particular at least 1.5 s. The time taken to nebulise a dose or to actuate the inhaler 1 is thus more than 0.7 s, more preferably at least about 1 s, more particularly more than 1.5 s.

The inhaler 1 has an add-on device 23 with a chamber 24 for intermediate storage of the aerosol 14 produced by the inhaler 1, as is shown in a schematic section in Fig. 3.

The chamber 24 is arranged or adapted to be arranged downstream of the delivery nozzle 12. It serves to receive and intermediately store the aerosol 14 produced by the inhaler 1.

The add-on device 23 or its chamber 24 is at least substantially cylindrical, elongate or conical in construction.

Preferably, the chamber 24 is of larger cross section than the mouthpiece 13 of the inhaler 1 and/or it widens out at least in parts towards the delivery end or free end of the add-on device 23. This ensures that the aerosol 14 strikes a wall of the chamber 24 over the smallest possible area. In this way it is possible to minimise the deposition or settling of the nebulised medicament preparation 2 on the wall of the chamber.

The chamber 24 preferably has a volume of more than 0.11, particularly more than 0.2 1, most preferably about 0.2 to 0.6 1. In particular, the add-on device 23 or the size of the chamber 24 is adapted to the inhaler 1 such that the aerosol 14 produced on actuation of the inhaler 1 can be at least substantially entirely received by the chamber 24 without the aerosol 14 or the nebulised medicament preparation 2 essentially being deposited or settling on the inner wall of the chamber.

The add-on device 23 in the embodiment shown preferably comprises a housing 25 which is in particular elongate and/or cylindrical in construction.

The add-on device 23 or its housing 25 is preferably at least substantially rigid in construction. However, the add-on device 23, the chamber 24 or the housing 25 may theoretically also be flexible, inflatable and/or telescopic in construction, in order to minimise the space taken up when not in use and/or for transportation purposes, in particular.

The add-on device 23 or the housing 25 has a connecting member 26 for connecting to the mouthpiece 13.

Preferably, the add-on device 23 or the connecting member 26 can be fitted onto the mouthpiece 13, particularly by a clamping effect, and/or can be released again from the inhaler 1 or mouthpiece 13. However, the add-on device 23 may if necessary be connected or connectable to the inhaler 1 in a fixed or non-removable manner.

The mouthpiece 13 has at least one supply air opening 15. At least one supply air opening 15 remains open when the add-on device 23 has been attached, particularly fitted on, as shown in Fig. 3. This may be achieved for example by corresponding conical design of the mouthpiece 13 and a complementary design of the connecting member 26, by a stop (not shown) and/or other design features.

Preferably, the add-on device 23 is not rotatable relative to the inhaler 1. This is achieved in the embodiment by designing the mouthpiece 13 of the inhaler 1 with a non-circular but preferably oval outer contour to which the connecting member 26 is matched accordingly. However, other design solutions are also possible.

The add-on device 23 or housing 25 preferably comprises a delivery member 27 for delivering the aerosol 14. The delivery member 27 is preferably arranged on the end of the chamber 24 or of the housing 25 which is opposite the connecting member 26.

Particularly preferably, the connecting member 26 and the delivery member 27 are formed in one piece with the housing 25. However, other design solutions are also possible.

Preferably, the chamber 24 or the housing 25 is at least partly or totally transparent in construction. This assists cleaning, in particular.

The add-on device 23 preferably has, at the delivery end, a valve 28 for preventing air from flowing back into the chamber 24. In this way it is possible to prevent air from flowing into the chamber 24 as the user breathes out, which would force out the aerosol 14 through the attached mouthpiece 13 and the supply air openings 15, for example.

The valve 28 is preferably a non-return valve.

Particularly preferably, the valve 28 is incorporated in the connecting member 27. Particularly preferably, the valve 28 can be detached from the add-on device 23 or the housing 25, for example for cleaning purposes.

Alternatively or in addition to the valve 28 the proposed inhaler I may also have a valve device (not shown) in the region of the mouthpiece 13 and/or the supply air opening or openings 15 to prevent air from flowing back out of the chamber 24 through the mouthpiece 13 and out through the supply air opening or openings 15.

Alternatively or in addition, the add-on device 23 may also have an additional valve device (not shown), for example between the connecting member 26 and the mouthpiece 13, to allow air to flow into the chamber 24 but prevent it from flowing out.

Preferably, the add-on device 23 comprises, alternatively or in addition, at least one valve 32 for blowing out the exhaled air, as schematically shown in Fig. 3. In particular, Fig. 3 shows two such valves 32 in the opened state. Valve flaps are lifted away from the associated outlet openings. The at least one valve 32 is preferably at the delivery end and in particular is arranged on the delivery member 27. However, other design solutions are also possible.

When a user (not shown) breathes in, the valve 28 opens, as shown by broken lines in Fig. 3. The valves 32 are closed. The aerosol 14 is sucked out of the chamber 24 and emitted through the delivery member 27. As the user breathes out, the valve 28 closes or is closed. The valves 32 open and allows the exhaled air to be blown out without this air flowing into the chamber 24 or affecting the aerosol 14.

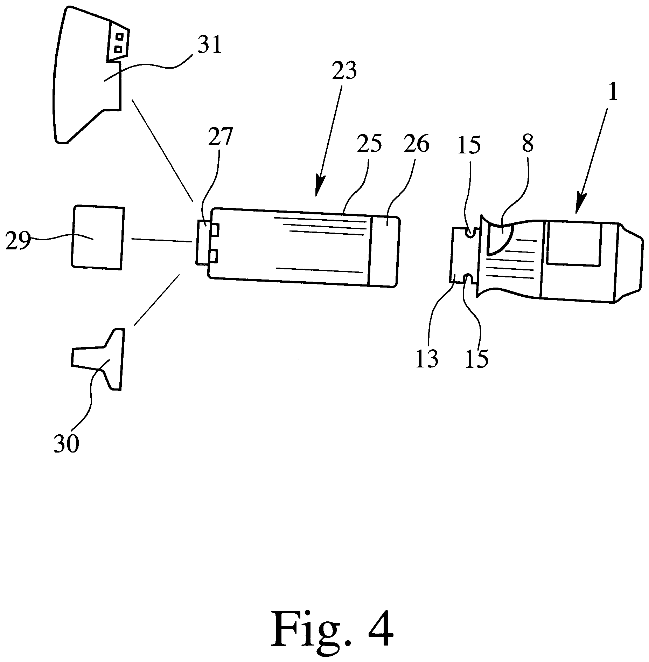

The add-on device 23 or its delivery member 27 may be equipped at the delivery end, preferably with an add-on mouthpiece 29, a tube 30 and/or a face mask 31, as shown by way of example in Fig. 4. In particular, different end pieces such as the add-on mouthpiece 29, the tube 30 and/or the face mask 31 can be selectively attached to the add-on device 23 or its delivery member 27, most preferably by fitting on.

Tests have shown that the proposed use of the add-on device 23 with the inhaler 1, i.e. the proposed intermediate storage of the aerosol 14 produced by the inhaler 1 in a sufficiently large chamber 24 may substantially contribute to a higher proportion of the active substance being received in the lungs on inhalation, even where there are problems of co-ordination.

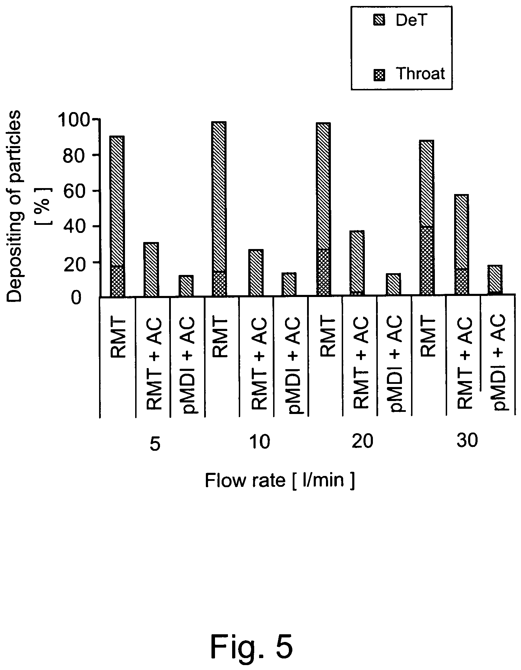

The enclosed Fig. 5 illustrates the proportion of active substance which is supplied for the lungs as a whole (DeT) and the proportion of active substance deposited in the throat (Throat) as a function of the overall dosage amount for different inhalers.

The vertical axis shows the percentage amount of the respective dose which is actually delivered, while DeT shows the proportion which is made available to the lungs on inhalation, and Throat indicates the proportion which is deposited in the throat. The deposition of active substance was determined on the basis of the declared content using a Finlay throat model. An inhaled volume of 0.5 1 in all was taken as the basis.

The tests were carried out for different apparatus or combinations of apparatus and at different flow rates, as plotted on the horizontal axis. RMT indicates the results of the inhaler 1 without the add-on device 23. RMT+AC indicates the results when the inhaler 1 is combined with the add-on device 23. pMDI+AC gives the results when a conventional MDI is combined with the add-on device 23. The numbers 5, 10, 20 and 30 each indicate the flow rate in 1/min.

Fig. 5 shows that the use of the add-on device 23 leads to a reduction in the throat deposits. This is favourable for paediatric applications as active substance deposited in the throat generally does not contribute to the therapy but instead often leads to (systemic) side effects. The use of the add-on device 23 is good as a slight deposit in the throat can only be detected above a flow rate of 20 1/min when the add-on device 23 is used. At all the flow rates, the deposition in the throat with the proposed combination of the inhaler 1 (SMI) with the add-on device 23 is lower than that of the inhaler 1 (SMI) on its own. The DeT can be correlated with the possible therapeutic effect. The proposed combination of the inhaler 1 (SMI) with the add-on device 23 always has a higher DeT than a conventional MDI with the add-on device 23. The loss of DeT by the use of the add-on device 23 compared with the proposed inhaler 1 without the add-on device 23 is detectable but must be estimated as being substantially lower than in a conventional MDI. Therefore, with the proposed solution, a higher efficacy or a higher proportion of active substance reaching the lungs can be assumed.

The preferred embodiment of the inhaler 1 is disclosed in WO 91/14468 A1 and WO 97/12687 A1.

In contrast to freestanding appliances or the like, the proposed inhaler 1 is designed to be portable and in particular is a mobile hand-held device.

By virtue of its cylindrical shape and handy size of less than 9 to 15 cm long and 2 to 4 cm wide, the inhaler 1 can be carried by the patient at all times. The nebuliser sprays a defined volume of the medicament preparation 2 by the application of high pressure through small nozzles, so as to form inhalable aerosols 14.

The proposed inhaler 1 operates purely mechanically, in particular. The pressure generator 5 for conveying and nebulising the medicament preparation 2 is constructed as a pump and/or operating mechanically.

The proposed inhaler 1 is designed in particular for the brief nebulisation of the medicament preparation 2, for example for one to two breaths. However, it may also be designed or used for longer or continuous nebulisation.

Some preferred ingredients, compounds and/or formulations of the medicament preparation 2 are listed below.

The compounds listed below may be used in the device according to the invention on their own or in combination. In the compounds mentioned below, W is a pharmacologically active substance and is selected (for example) from among the betamimetics, anticholinergics, corticosteroids, PDE4-inhibitors, LTD4-antagonists, EGFR-inhibitors, dopamine agonists, HI-antihistamines, PAF-antagonists and PI3-kinase inhibitors. Moreover, double or triple combinations of **W** may be combined and used in the device according to the invention. Combinations of **W** might be, for example:
- **W** denotes a betamimetic, combined with an anticholinergic, corticosteroid, PDE4-inhibitor, EGFR-inhibitor or LTD4-antagonist,
- **W** denotes an anticholinergic, combined with a betamimetic, corticosteroid, PDE4-inhibitor, EGFR-inhibitor or LTD4-antagonist,
- **W** denotes a corticosteroid, combined with a PDE4-inhibitor, EGFR-inhibitor or LTD4-antagonist
- **W** denotes a PDE4-inhibitor, combined with an EGFR-inhibitor or LTD4-antagonist
- **W** denotes an EGFR-inhibitor, combined with an LTD4-antagonist.

The compounds used as betamimetics are preferably compounds selected from among albuterol, arformoterol, bambuterol, bitolterol, broxaterol, carbuterol, clenbuterol, fenoterol, formoterol, hexoprenaline, ibuterol, isoetharine, isoprenaline, levosalbutamol, mabuterol, meluadrine, metaproterenol, orciprenaline, pirbuterol, procaterol, reproterol, rimiterol, ritodrine, salmefamol, salmeterol, soterenol, sulphonterol, terbutaline, tiaramide, tolubuterol, zinterol, CHF-1035, HOKU-81, KUL-1248 and
- 3-(4-{6-[2-hydroxy-2-(4-hydroxy-3-hydroxymethyl-phenyl)-ethylamino]-hexyloxy}-butyl)-benzyl-sulphonamide
- 5-[2-(5,6-diethyl-indan-2-ylamino)-1-hydroxy-ethyl]-8-hydroxy-1H-quinolin-2-one
- 4-hydroxy-7-[2-{[2-{[3-(2-phenylethoxy)propyl]sulphonyl}ethyl]-amino}ethyl]-2(3H)-benzothiazolone
- 1-(2-fluoro-4-hydroxyphenyl)-2-[4-(1-benzimidazolyl)-2-methyl-2-butylamino] ethanol
- 1-[3-(4-methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino} ethanol
- 5-hydroxy-8-(1-hydroxy-2-isopropylaminobutyl)-2H-1,4-benzoxazin-3-(4H)-one
- 1-(4-amino-3-chloro-5-trifluoromethylphenyl)-2-tert.-butylamino)ethanol
- 6-hydroxy-8-{1-hydroxy-2-[2-(4-methoxy-phenyl)-1,1-dimethyl-ethylamino]-ethyl}-4H-benzo[1,4]oxazin-3-one
- 6-hydroxy-8-{1-hydroxy-2-[2-(ethyl 4-phenoxy-acetate)-1,1-dimethyl-ethylamino]-ethyl}-4H-benzo[1,4]oxazin-3-one
- 6-hydroxy-8-{1-hydroxy-2-[2-(4-phenoxy-acetic acid)-1,1-dimethyl-ethylamino]-ethyl)-4H-benzo[1,4]oxazin-3-one
- 8-{2-[1,1-dimethyl-2-(2,4,6-trimethylphenyl)-ethylamino]-1-hydroxy-ethyl}-6-hydroxy-4H-benzo[1,4]oxazin-3 -one
- 6-hydroxy-8-{1-hydroxy-2- [2-(4-hydroxy-phenyl)-1,1-dimethyl-ethylamino]-ethyl}-4H-benzo[1,4]oxazin-3-one
- 6-hydroxy-8-{1-hydroxy-2-[2-(4-isopropyl-phenyl)-1.1dimethyl-ethylamino]-ethyl}-4H-benzo[1,4]oxazin-3-one
- 8-{2-[2-(4-ethyl-phenyl)-1,1-dimethyl-ethylamino]-1-hydroxy-ethyl}-6-hydroxy-4H-benzo[1 ,4]oxazin-3-one
- 8-(2-[2-(4-ethoxy-phenyl)-1,1-dimethyl-ethylamino]-1-hydroxy-ethyl}-6-hydroxy-4H-benzo[1,4]oxazin-3-one
- 4-(4-{2-[2-hydroxy-2-(6-hydroxy-3-oxo-3,4-dihydro-2H-benzo[1,4]oxazin-8-yl)-ethylamino]-2-methyl-propyl}-phenoxy)-butyric acid
- 8-{2-[2-(3.4-difluoro-phenyl)-1,1-dimethyl-ethylamino]-1-hydroxy-ethyl}-6-hydroxy-4H-benzo[1,4]oxazin-3-one
- 1-(4-ethoxy-carbonylamino-3-cyano-5-fluorophenyl)-2-(tert-butylamino)ethanol
- 2-hydroxy-5-(1-hydroxy-2-{2-[4-(2-hydroxy-2-phenyl-ethylamino)-phenyl]-ethylamino}-ethyl)-benzaldehyde
- N-[2-hydroxy-5-(1-hydroxy-2-{2-[4-(2-hydroxy-2-phenyl-ethylamino)-phenyl]-ethylamino} -ethyl)-phenyl]-formamide
- 8-hydroxy-5-(1-hydroxy-2-{2-[4-(6-methoxy-biphenyl-3-ylamino)-phenyl]-ethylamino}-ethyl)-1H-quinolin-2-one
- 8-hydroxy-5-[1-hydroxy-2-(6-phenethylamino-hexylamino)-ethyl]-1H-quinolin-2-one
- 5- [2-(2- {4- [4-(2-amino-2-methyl-propoxy)-phenylamino]-phenyl} -ethylamino)-1-hydroxy-ethyl]-8-hydroxy-1H-quinolin-2-one
- [3-(4-{6-[2-hydroxy-2-(4-hydroxy-3-hydroxymethyl-phenyl)-ethylamino]-hexyloxy}-butyl)-5-methyl-phenyl]-urea
- 4-(2- {6-[2-(2,6-dichloro-benzyloxy)-ethoxy]-hexylamino}-1-hydroxy-ethyl)-2-hydroxymethyl-phenol
- 3-(4-{6-[2-hydroxy-2-(4-hydroxy-3-hydroxymethyl-phenyl)-ethylamino]-hexyloxy} -butyl)-benzylsulphonamide
- 3-(3-{7-[2-hydroxy-2-(4-hydroxy-3-hydroxymethyl-phenyl)-ethylamino]-heptyloxy} -propyl)-benzylsulphonamide
- 4-(2-{6-[4-(3-cyclopentanesulphonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamide
optionally in the form of the racemates, enantiomers, diastereomers thereof and optionally in the form of the pharmacologically acceptable acid addition salts, solvates or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrobenzoate and hydro-p-toluenesulphonate.

The anticholinergics used are preferably compounds selected from among the tiotropium salts, preferably the bromide salt, oxitropium salts, preferably the bromide salt, flutropium salts, preferably the bromide salt, ipratropium salts, preferably the bromide salt, glycopyrronium salts, preferably the bromide salt, trospium salts, preferably the chloride salt, tolterodine. In the above-mentioned salts the cations are the pharmacologically active constituents. As anions the above-mentioned salts may preferably contain the chloride, bromide, iodide, sulphate, phosphate, methanesulphonate, nitrate, maleate, acetate, citrate, fumarate, tartrate, oxalate, succinate, benzoate or p-toluenesulphonate, while chloride, bromide, iodide, sulphate, methanesulphonate or p-toluenesulphonate are preferred as counter-ions.

Of all the salts the chlorides, bromides, iodides and methanesulphonates are particularly preferred.

Other preferred anticholinergics are selected from among the salts of formula **AC-1** 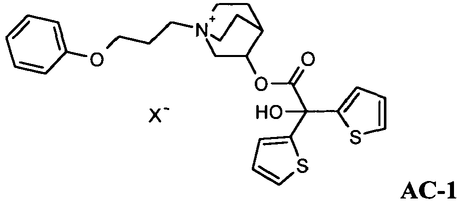 wherein X⁻ denotes an anion with a single negative charge, preferably an anion selected from among the fluoride, chloride, bromide, iodide, sulphate, phosphate, methanesulphonate, nitrate, maleate, acetate, citrate, fumarate, tartrate, oxalate, succinate, benzoate and p-toluenesulphonate, preferably an anion with a single negative charge, particularly preferably an anion selected from among the fluoride, chloride, bromide, methanesulphonate and p-toluenesulphonate, particularly preferably bromide, optionally in the form of the racemates, enantiomers or hydrates thereof. Of particular importance are those pharmaceutical combinations which contain the enantiomers of formula **AC-1-en** 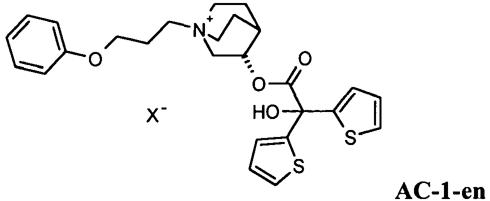 wherein X⁻ may have the above-mentioned meanings. Other preferred anticholinergics are selected from the salts of formula **AC-2** 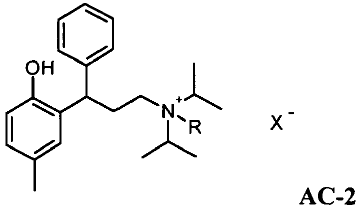 wherein R denotes either methyl or ethyl and wherein X⁻ may have the above-mentioned meanings. In an alternative embodiment the compound of formula **AC-2** may also be present in the form of the free base **AC-2-base.**

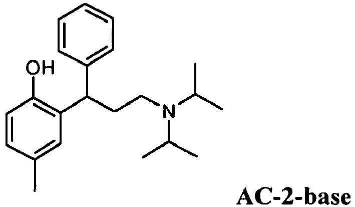

Other specified compounds are:
- tropenol 2,2-diphenylpropionate methobromide,
- scopine 2,2-diphenylpropionate methobromide,
- scopine 2-fluoro-2,2-diphenylacetate methobromide,
- tropenol 2-fluoro-2,2-diphenylacetate methobromide;
- tropenol 3,3',4,4'-tetrafluorobenzilate methobromide,
- scopine 3,3',4,4'-tetrafluorobenzilate methobromide,
- tropenol 4,4'-difluorobenzilate methobromide,
- scopine 4,4'-difluorobenzilate methobromide,
- tropenol 3,3'-difluorobenzilate methobromide,
- scopine 3,3'- difluorobenzilate methobromide;
- tropenol 9-hydroxy-fluorene-9-carboxylate methobromide;
- tropenol 9-fluoro-fluorene-9-carboxylate methobromide;
- scopine 9-hydroxy-fluorene-9-carboxylate methobromide;
- scopine 9-fluoro-fluorene-9-carboxylate methobromide;
- tropenol 9-methyl-fluorene-9-carboxylate methobromide;
- scopine 9-methyl-fluorene-9-carboxylate methobromide;
- cyclopropyltropine benzilate methobromide;
- cyclopropyltropine 2,2-diphenylpropionate methobromide;
- cyclopropyltropine 9-hydroxy-xanthene-9-carboxylate methobromide;
- cyclopropyltropine 9-methyl-fluorene-9-carboxylate methobromide;
- cyclopropyltropine 9-methyl-xanthene-9-carboxylate methobromide;
- cyclopropyltropine 9-hydroxy-fluorene-9-carboxylate methobromide;
- cyclopropyltropine methyl 4,4'-difluorobenzilate methobromide.
- tropenol 9-hydroxy-xanthene-9-carboxylate methobromide;
- scopine 9-hydroxy-xanthene-9-carboxylate methobromide;
- tropenol 9-methyl-xanthene-9-carboxylate methobromide;
- scopine 9-methyl-xanthene-9-carboxylate methobromide;
- tropenol 9-ethyl-xanthene-9-carboxylate methobromide;
- tropenol 9-difluoromethyl-xanthene-9-carboxylate methobromide;
- scopine 9-hydroxymethyl-xanthene-9-carboxylate methobromide,

The above-mentioned compounds may also be used as salts within the scope of the present invention, wherein instead of the methobromide the metho-X salts are used, wherein X may have the meanings given hereinbefore for X⁻.

As corticosteroids it is preferable to use compounds selected from among beclomethasone, betamethasone, budesonide, butixocort, ciclesonide, deflazacort, dexamethasone, etiprednol, flunisolide, fluticasone, loteprednol, mometasone, prednisolone, prednisone, rofleponide, triamcinolone, RPR-106541, NS-126, ST-26 and
- (S)-fluoromethyl 6,9-difluoro-17-[(2-furanylcarbonyl)oxy]-11-hydroxy-16-methyl-3-oxo-androsta-1,4-diene-17-carbothionate
- (S)-(2-oxo-tetrahydro-furan-3S-yl)6,9-difluoro-11-hydroxy-16-methyl-3-oxo-17-propionyloxy-androsta-1,4-diene-17-carbothionate,
- cyanomethyl 6α,9α-difluoro-11β-hydroxy-16α-methyl-3-oxo-17α-(2,2,3,3-tertamethylcyclopropylcarbonyl)oxy-androsta-1,4-diene-17β-carboxylate optionally in the form of the racemates, enantiomers or diastereomers thereof and optionally in the form of the salts and derivatives thereof, the solvates and/or hydrates thereof. Any reference to steroids includes a reference to any salts or derivatives, hydrates or solvates thereof which may exist. Examples of possible salts and derivatives of the steroids may be: alkali metal salts, such as for example sodium or potassium salts, sulphobenzoates, phosphates, isonicotinates, acetates, dichloroacetates, propionates, dihydrogen phosphates, palmitates, pivalates or furoates.

PDE4-inhibitors which may be used are preferably compounds selected from among enprofyllin, theophyllin, roflumilast, ariflo (cilomilast), tofimilast, pumafentrin, lirimilast, arofyllin, atizoram, D-4418, Bay-198004, BY343, CP-325.366, D-4396 (Sch-351591), AWD-12-281 (GW-842470), NCS-613, CDP-840, D-4418, PD-168787, T-440, T-2585, V-11294A, C1-1018, CDC-801, CDC-3052, D-22888, YM-58997, Z-15370 and
- N-(3,5-dichloro-1-oxo-pyridin-4-yl)-4-difluoromethoxy-3-cyclopropylmethoxybenzamide
- (-)p-[(4*a*R*,10*b*S*)-9-ethoxy-1 ,2,3,4,4a,10b-hexahydro-8-methoxy-2-methylbenzo[s] [1,6]naphthyridin-6-yl]-N,N-diisopropylbenzamide
- (R)-(+)-1-(4-bromobenzyl)-4-[(3-cyclopentyloxy)-4-methoxyphenyl]-2-pyrrolidone
- 3-(cyclopentyloxy-4-methoxyphenyl)-1-(4-N'-[N-2-cyano-S-methyl-isothioureido]benzyl)-2-pyrrolidone
- cis[4-cyano-4-(3-cyclopentyloxy-4-methoxyphenyl)cyclohexane-1-carboxylic acid]
- 2-carbomethoxy-4-cyano-4-(3-cyclopropylmethoxy-4-difluoromethoxyphenyl)cyclohexan-1-one
- cis[4-cyano-4-(3-cyclopropylmethoxy-4-difluoromethoxyphenyl)cyclohexan-1-ol]
- (R)-(+)-ethyl[4-(3-cyclopentyloxy-4-methoxyphenyl)pyrrolidin-2-ylidene]acetate
- (S)-(-)-ethyl[4-(3-cyclopentyloxy-4-methoxyphenyl)pyrrolidin-2-ylidene]acetate
- 9-cyclopentyl-5,6-dihydro-7-ethyl-3-(2-thienyl)-9*H*-pyrazolo[3,4-c]-1,2,4-triazolo[4,3-a]pyridine
- 9-cyclopentyl-5,6-dihydro-7-ethyl-3-(*tert*-butyl)-9*H*-pyrazolo[3,4-c]-1,2,4-triazolo[4,3-a]pyridine
optionally in the form of the racemates, enantiomers or diastereomers thereof and optionally in the form of the pharmacologically acceptable acid addition salts thereof, the solvates and/or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrobenzoate and hydro-p-toluenesulphonate.

The LTD4-antagonists used are preferably compounds selected from among montelukast, pranlukast, zafirlukast, MCC-847 (ZD-3523), MN-001, MEN-91507 (LM-1507), VUF-5078, VUF-K-8707, L-733321 and
- 1-(((R)-(3-(2-(6,7-difluoro-2-quinolinyl)ethenyl)phenyl)-3-(2-(2-hydroxy-2-propyl)phenyl)thio)methylcyclopropane-acetic acid,
- 1-(((1(R)-3(3-(2-(2,3-dichlorothieno[3,2-b]pyridin-5-yl)-(E)-ethenyl)phenyl)-3-(2-(1-hydroxy-1-methylethyl)phenyl)propyl)thio)methyl)cyclopropaneacetic acid
- [2-[[2-(4-tert-butyl-2-thiazolyl)-5-benzofuranyl]oxymethyl]phenyl]acetic acid optionally in the form of the racemates, enantiomers or diastereomers thereof and optionally in the form of the pharmacologically acceptable acid addition salts, solvates and/or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrobenzoate and hydro-p-toluenesulphonate. By salts or derivatives which the LTD4-antagonists may optionally be capable of forming are meant, for example: alkali metal salts, such as for example sodium or potassium salts, alkaline earth metal salts, sulphobenzoates, phosphates, isonicotinates, acetates, propionates, dihydrogen phosphates, palmitates, pivalates or furoates.

EGFR-inhibitors which may be used are preferably compounds selected from among cetuximab, trastuzumab, ABX-EGF, Mab ICR-62 and
- 4-[(3-chloro-4-fluorophenyl)amino]-6-{[4-(morpholin-4-yl)-1-oxo-2-buten-1-yl]-amino}-7-cyclopropylmethoxy-quinazoline
- 4-[(3-chloro-4-fluorophenyl)amino]-6-{[4-(N,N-diethylamino)-1-oxo-2-buten-1-yl]amino}-7-cyclopropylmethoxy-quinazoline
- 4-[(3-chloro-4-fluorophenyl)amino]-6-{[4-(N,N-dimethylamino)-1-oxo-2-buten-1-yl]amino}-7-cyclopropylmethoxy-quinazoline
- 4-[(R)-(1-phenyl-ethyl)amino]-6-{[4-(morpholin-4-yl)-1-oxo-2-buten-1-yl]-amino}-7-cyclopentyloxy-quinazoline
- 4-[(3-chloro-4-fluoro-phenyl)amino]-6-{[4-((R)-6-methyl-2-oxo-morpholin-4-yl)-1-oxo-2-buten-1-yl]amino}-7-cyclopropylmethoxy-quinazoline
- 4-[(3-chloro-4-fluoro-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-quinazoline
- 4-[(3-chloro-4-fluoro-phenyl)amino]-6-{[4-((R)-2-methoxymethyl-6-oxo-morpholin-4-yl)-1-oxo-2-buten-1-yl]amino}-7-cyclopropylmethoxy-quinazoline
- 4-[(3-chloro-4-fluoro-phenyl)amino]-6-[2-((S)-6-methyl-2-oxo-morpholin-4-yl)-ethoxy]-7-methoxy-quinazoline
- 4-[(3-chloro-4-fluorophenyl)amino]-6-({4-[N-(2-methoxy-ethyl)-N-methyl-amino]-1-oxo-2-buten-1-yl}amino)-7-cyclopropylmethoxy-quinazoline
- 4-[(3-chloro-4-fluorophenyl)amino]-6-{[4-(N,N-dimethylamino)-1-oxo-2-buten-1-yl]amino}-7-cyclopentyloxy-quinazoline
- 4-[(R)-(1-phenyl-ethyl)amino]-6-{[4-(N,N-to-(2-methoxy-ethyl)-amino)-1-oxo-2-buten-1-yl] amino}-7-cyclopropylmethoxy-quinazoline
- 4-[(R)-(1-phenyl-ethyl)amino]-6-({4-[N-(2-methoxy-ethyl)-N-ethyl-amino]-1-oxo-2-buten-1-yl}amino)-7-cyclopropylmethoxy-quinazoline
- 4-[(R)-(1-phenyl-ethyl)amino]-6-({4-[N-(2-methoxy-ethyl)-N-methyl-amino]-1-oxo-2-buten-1-yl}amino)-7-cyclopropylmethoxy-quinazoline
- 4-[(R)-(1-phenyl-ethyl)amino]-6-({4-[N-(tetrahydropyran-4-yl)-N-methyl-amino]-1-oxo-2-buten-1-yl)amino)-7-cyclopropyltnethoxy-quinazoline
- 4-[(3-chloro-4-fluorophenyl)amino]-6-{[4-(N,N-dimethylamino)-1-oxo-2-buten-1-yl]amino}-7-((R)-tetrahydrofuran-3-yloxy)-quinazoline
- 4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline
- 4-[(3-chloro-4-fluorophenyl)amino]-6-({4-[N-(2-methoxy-ethyl)-N-methyl-amino]-1-oxo-2-buten-1-yl}amino)-7-cyclopentyloxy-quinazoline
- 4-[(3-chloro-4-fluorophenyl)amino]-6-{[4-(N-cyclopropyl-N-methyl-amino)-1-oxo-2-buten-1-yl]amino}-7-cyclopentyloxy-quinazoline
- 4-[(3-chloro-4-fluorophenyl)amino]-6-{[4-(N,N-dimethylamino)-1-oxo-2-buten-1-yl]amino}-7-[(R)-(tetrahydrofuran-2-yl)methoxy]-quinazoline
- 4-[(3-chloro-4-fluorophenyl)amino]-6-{[4-(N,N-dimethylamino)-1-oxo-2-buten-1-yl]amino}-7-[(S)-(tetrahydrofuran-2-yl)methoxy]-quinazoline
- 4-[(3-ethynyl-phenyl)amino]-6.7-to-(2-methoxy-ethoxy)-quinazoline
- 4-[(3-chloro-4-fluorophenyl)amino]-7-[3-(morpholin-4-yl)-propyloxy]-6-[(vinyl-carbonyl)amino]-quinazoline
- 4-[(R)-(1-phenyl-ethyl)amino]-6-(4-hydroxy-phenyl)-7H-pyrrolo[2,3-d]pyrimidine
- 3-cyano-4-[(3-chloro-4-fluorophenyl)amino]-6-{[4-(N,N-dimethylamino)-1-oxo-2-buten-1-yl]amino}-7-ethoxy-quinoline
- 4-{[3-chloro-4-(3-fluoro-benzyloxy)-phenyl]amino}-6-(5-{[(2-methanesulphonyl-ethyl)amino]methyl} -furan-2-yl)quinazoline
- 4-[(R)-(1-phenyl-ethyl)amino]-6-{[4-((R)-6-methyl-2-oxo-morpholin-4-yl)-1-oxo-2-buten-1-yl]amino}-7-methoxy-quinazoline
- 4-[(3-chloro-4-fluorophenyl)amino]-6-{[4-(morpholin-4-yl)-1-oxo-2-buten-1-yl]-amino}-7-[(tetrahydrofuran-2-yl)methoxy]-quinazoline
- 4-[(3-chloro-4-fluorophenyl)amino]-6-({4-[N,N-to-(2-methoxy-ethyl)-amino]-1-oxo-2-buten-1-yl}amino)-7-[(tetrahydrofuran-2-yl)methoxy]-quinazoline
- 4-[(3-ethynyl-phenyl)amino]-6-{[4-(5.5-dimethyl-2-oxo-morpholin-4-yl)-1-oxo-2-buten-1-yl]amino}-quinazoline
- 4-[(3-chloro-4-fluoro-phenyl)amino]-6-[2-(2,2-dimethyl-6-oxo-morpholin-4-yl)-ethoxy]-7-methoxy-quinazoline
- 4-[(3-chloro-4-fluoro-phenyl)amino]-6-[2-(2,2-dimethyl-6-oxo-morpholin-4-yl)-ethoxy]-7-[(R)-(tetrahydrofuran-2-yl)methoxy]-quinazoline
- 4-[(3-chloro-4-fluoro-phenyl)amino]-7-[2-(2,2-dimethyl-6-oxo-morpholin-4-yl)-ethoxy]-6-[(S)-(tetrahydrofuran-2-yl)methoxy]-quinazoline
- 4-[(3-chloro-4-fluoro-phenyl)amino]-6-{2-[4-(2-oxo-morpholin-4-yl)-piperidin-1-yl]-ethoxy}-7-methoxy-quinazoline
- 4-[(3-chloro-4-fluoro-phenyl)amino]-6-[1-(tert.-butyloxycarbonyl)-piperidin-4-yloxy]-7-methoxy-quinazoline
- 4-[(3-chloro-4-fluoro-phenyl)amino]-6-(trans-4-amino-cyclohexan-1-yloxy)-7-methoxy-quinazoline
- 4-[(3-chloro-4-fluoro-phenyl)amino]-6-(trans-4-methanesulphonylamino-cyclohexan-1-yloxy)-7-methoxy-quinazoline
- 4-[(3-chloro-4-fluoro-phenyl)amino]-6-(tetrahydropyran-3-yloxy)-7-methoxy-quinazoline
- 4-[(3-chloro-4-fluoro-phenyl)amino]-6-(1-methyl-piperidin-4-yloxy)-7-methoxy-quinazoline
- 4-[(3-chloro-4-fluoro-phenyl)amino]-6-{1-[(morpholin-4-yl)carbonyl]-piperidin-4-yloxy}-7-methoxy-quinazoline
- 4-[(3-chloro-4-fluoro-phenyl)amino]-6-{1-[(methoxymethyl)carbonyl]-piperidin-4-yloxy}-7-methoxy-quinazoline
- 4-[(3-chloro-4-fluoro-phenyl)amino]-6-(piperidin-3-yloxy)-7-methoxy-quinazoline
- 4-[(3-chloro-4-fluoro-phenyl)amino]-6-[1-(2-acetylamino-ethyl)-piperidin-4-yloxy]-7-methoxy-quinazoline
- 4-[(3-chloro-4-fluoro-phenyl)amino]-6-(tetrahydropyran-4-yloxy)-7-ethoxy-quinazoline
- 4-[(3-chloro-4-fluoro-phenyl)amino]-6-((S)-tetrahydrofuran-3-yloxy)-7-hydroxy-quinazoline
- 4-[(3-chloro-4-fluoro-phenyl)amino]-6-(tetrahydropyran-4-yloxy)-7-(2-methoxy-ethoxy)-quinazoline
- 4-[(3-chloro-4-fluoro-phenyl)amino]-6-{trans-4-[(dimethylamino)sulphonylamino]-cyclohexan-1-yloxy)-7-methoxy-quinazoline
- 4-[(3-chloro-4-fluoro-phenyl)amino]-6-{trans-4-[(morpholin-4-yl)carbonylamino]-cyclohexan-1-yloxy}-7-methoxy-quinazoline
- 4-[(3-chloro-4-fluoro-phenyl)amino]-6-{trans-4-[(morpholin-4-yl)sulphonylamino]-cyclohexan-1-yloxy}-7-methoxy-quinazoline
- 4-[(3-chloro-4-fluoro-phenyl)amino]-6-(tetrahydropyran-4-yloxy)-7-(2-acetylamino-ethoxy)-quinazoline
- 4-[(3-chloro-4-fluoro-phenyl)amino]-6-(tetrahydropyran-4-yloxy)-7-(2-methanesulphonylamino-ethoxy)-quinazoline
- 4-[(3-chloro-4-fluoro-phenyl)amino]-6-{1-[(piperidin-1-yl)carbonyl]-piperidin-4-yloxy}-7-methoxy-quinazoline
- 4-[(3-chloro-4-fluoro-phenyl)amino]-6-(1-aminocarbonylmethyl-piperidin-4-yloxy)-7-methoxy-quinazoline
- 4-[(3-chloro-4-fluoro-phenyl)amino]-6-(cis-4-{N-[(tetrahydropyran-4-yl)carbonyl]-N-methyl-amino}-cyclohexan-1-yloxy)-7-methoxy-quinazoline
- 4-[(3-chloro-4-fluoro-phenyl)amino]-6-(cis-4-{N-[(morpholin-4-yl)carbonyl]-N-methyl-amino}-cyclohexan-1-yloxy)-7-methoxy-quinazoline
- 4-[(3-chloro-4-fluoro-phenyl)amino]-6-(cis-4-{N-[(morpholin-4-yl)sulphonyl]-N-methyl-amino}-cyclohexan-1-yloxy)-7-methoxy-quinazoline
- 4-[(3-chloro-4-fluoro-phenyl)amino]-6-(trans-4-ethanesulphonylamino-cyclohexan-1-yloxy)-7-methoxy-quinazoline
- 4-[(3-chloro-4-fluoro-phenyl)amino]-6-(1-methanesulphonyl-piperidin-4-yloxy)-7-ethoxy-quinazoline
- 4-[(3-chloro-4-fluoro-phenyl)amino]-6-(1-methanesulphonyl-piperidin-4-yloxy)-7-(2-methoxy-ethoxy)-quinazoline
- 4-[(3-chloro-4-fluoro-phenyl)amino]-6-[1-(2-methoxy-acetyl)-piperidin-4-yloxy]-7-(2-methoxy-ethoxy)-quinazoline
- 4-[(3-chloro-4-fluoro-phenyl)amino]-6-(cis-4-acetylamino-cyclohexan-1-yloxy)-7-methoxy-quinazoline
- 4-[(3-ethynyl-phenyl)amino]-6-[1-(tert.-butyloxycarbonyl)-piperidin-4-yloxy]-7-methoxy-quinazoline
- 4-[(3-ethynyl-phenyl)amino]-6-(tetrahydropyran-4-yloxy]-7-methoxy-quinazoline
- 4-[(3-chloro-4-fluoro-phenyl)amino]-6-(cis-4-{N-[(piperidin-1-yl)carbonyl]-N-methyl-amino}-cyclohexan-1-yloxy)-7-methoxy-quinazoline
- 4-[(3-chloro-4-fluoro-phenyl)amino]-6-(cis-4-{N-[(4-methyl-piperazin-1-yl)carbonyl]-N-methyl-amino}-cyclohexan-1-yloxy)-7-methoxy-quinazoline
- 4-[(3-chloro-4-fluoro-phenyl)amino]-6-{cis-4-[(morpholin-4-yl)carbonylamino]-cyclohexan-1-yloxy}-7-methoxy-quinazoline
- 4-[(3-chloro-4-fluoro-phenyl)amino]-6-{1-[2-(2-oxopyrrolidin-1-yl)ethyl]-piperidin-4-yloxy}-7-methoxy-quinazoline
- 4-[(3-chloro-4-fluoro-phenyl)amino]-6-{1-[(morpholin-4-yl)carbonyl]-piperidin-4-yloxy}-7-(2-methoxy-ethoxy)-quinazoline
- 4-[(3-ethynyl-phenyl)amino]-6-(1-acetyl-piperidin-4-yloxy)-7-methoxy-quinazoline
- 4-[(3-ethynyl-phenyl)amino]-6-(1-methyl-piperidin-4-yloxy)-7-methoxy-quinazoline
- 4-[(3-ethynyl-phenyl)amino]-6-(1-methanesulphonyl-piperidin-4-yloxy)-7-methoxy-quinazoline
- 4-[(3-chloro-4-fluoro-phenyl)amino]-6-(1-methyl-piperidin-4-yloxy)-7(2-methoxy-ethoxy)-quinazoline
- 4-[(3-chloro-4-fluoro-phenyl)amino]-6-(1-isopropyloxycarbonyl-piperidin-4-yloxy)-7-methoxy-quinazoline
- 4-[(3-chloro-4-fluoro-phenyl)amino]-6-(cis-4-methylamino-cyclohexan-1-yloxy)-7-methoxy-quinazoline
- 4-[(3-chloro-4-fluoro-phenyl)amino]-6-{cis-4-[N-(2-methoxy-acetyl)-N-methyl-amino]-cyclohexan-1-yloxy}-7-methoxy-quinazoline
- 4-[(3-ethynyl-phenyl)amino]-6-(piperidin-4-yloxy)-7-methoxy-quinazoline
- 4-[(3-ethynyl-phenyl)amino]-6-[1-(2-methoxy-acetyl)-piperidin-4-yloxy]-7-methoxy-quinazoline
- 4-[(3-ethynyl-phenyl)amino]-6-{1-[(morpholin-4-yl)carbonyl]-piperidin-4-yloxy}-7-methoxy-quinazoline
- 4-[(3-chloro-4-fluoro-phenyl)amino]-6-{-[(cis-2,6-dimethyl-morpholin-4-yl)carbonyl]-piperidin-4-yloxy}-7-methoxy-quinazoline
- 4-[(3-chloro-4-fluoro-phenyl)amino]-6-{1-[(2-methyl-morpholin-4-yl)carbonyl]-piperidin-4-yloxy}-7-methoxy-quinazoiine
- 4-[(3-chloro-4-fluoro-phenyl)amino]-6-{1-[(S,S)-(2-oxa-5-aza-bicyclo[2,2,1]hept-5-yl)carbonyl]-piperidin-4-yloxy}-7-methoxy-quinazoline
- 4-[(3-chloro-4-fluoro-phenyl)amino]-6-{1-[(N-methyl-N-2-methoxyethyl-amino)carbonyl]-piperidin-4-yloxy}-7-methoxy-quinazoline
- 4-[(3-chloro-4-fluoro-phenyl)amino]-6-(1-ethyl-piperidin-4-yloxy)-7-methoxy-quinazoline
- 4-[(3-chloro-4-fluoro-phenyl)amino]-6-{1-[(2-methoxyethyl)carbonyl]-piperidin-4-yloxy}-7-methoxy-quinazoline
- 4-[(3-chloro-4-fluoro-phenyl)amino]-6-{1-[(3-methoxypropyl-amino)-carbonyl]-piperidin-4-yloxy}-7-methoxy-quinazoline
- 4-[(3-chloro-4-fluoro-phenyl)amino]-6-[cis-4-(N-methanesulphonyl-N-methyl-amino)-cyclohexan-1-yloxy]-7-methoxy-quinazoline
- 4-[(3-chloro-4-fluoro-phenyl)amino]-6-[cis-4-(N-acetyl-N-methyl-amino)-cyclohexan-1-yloxy]-7-methoxy-quinazoline
- 4-[(3-chloro-4-fluoro-phenyl)amino]-6-(trans-4-methylamino-cyclohexan-1-yloxy)-7-methoxy-quinazoline
- 4-[(3-chloro-4-fluoro-phenyl)amino]-6-[trans-4-(N-methanesulphonyl-N-methyl-amino)-cyclohexan-1-yloxy]-7-methoxy-quinazoline
- 4-[(3-chloro-4-fluoro-phenyl)amino]-6-(trans-4-dimethylamino-cyclohexan-1-yloxy)-7-methoxy-quinazoline
- 4-[(3-chloro-4-fluoro-phenyl)amino]-6-(trans-4-{N-[(morpholin-4-yl)carbonyl]-N-methyl-amino}-cyclohexan-1-yloxy)-7-methoxy-quinazoline
- 4-[(3-chloro-4-fluoro-phenyl)amino]-6-[2-(2,2-dimethyl-6-oxo-morpholin-4-yl)-ethoxy]-7-[(S)-(tetrahydrofuran-2-yl)methoxy]-quinazoline
- 4-[(3-chloro-4-fluoro-phenyl)amino]-6-(1-methanesulphonyl-piperidin-4-yloxy)-7-methoxy-quinazoline
- 4-[(3-chloro-4-fluoro-phenyl)amino]-6-(1-cyano-piperidin-4-yloxy)-7-methoxy-quinazoline
optionally in the form of the racemates, enantiomers, diastereomers thereof and optionally in the form of the pharmacologically acceptable acid addition salts, solvates or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrobenzoate and hydro-p-toluenesulphonate.

The dopamine agonists used are preferably compounds selected from among bromocriptin, cabergoline, alpha-dihydroergocryptine, lisuride, pergolide, pramipexol, roxindol, ropinirol, talipexol, tergurid and viozan, optionally in the form of the racemates, enantiomers, diastereomers thereof and optionally in the form of the pharmacologically acceptable acid addition salts, solvates or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydrooxalate, hydrosuccinate, hydrobenzoate and hydro-p-toluenesulphonate.

HI-Antihistamines which may be used are preferably compounds selected from among epinastine, cetirizine, azelastine, fexofenadine, levocabastine, loratadine, mizolastine, ketotifen, emedastine, dimetindene, clemastine, bamipine, cexchlorpheniramine, pheniramine, doxylamine, chlorophenoxamine, dimenhydrinate, diphenhydramine, promethazine, ebastine, desloratidine and meclozine, optionally in the form of the racemates, enantiomers, diastereomers thereof and optionally in the form of the pharmacologically acceptable acid addition salts, solvates or hydrates thereof. According to the invention the acid addition salts of the betamimetics are preferably selected from among the hydrochloride, hydrobromide, hydriodide, hydrosulphate, hydrophosphate, hydromethanesulphonate, hydronitrate, hydromaleate, hydroacetate, hydrocitrate, hydrofumarate, hydrotartrate, hydroxalate, hydrosuccinate, hydrobenzoate and hydro-p-toluenesulphonate.

In addition, inhalable macromolecules as disclosed in EP 1 003 478 A1 or CA 2297174 A1 may also be used.

In addition, the compound may be selected from among the ergot alkaloid derivatives, the triptans, the CGRP-inhibitors, the phosphodiesterase-V inhibitors, optionally in the form of the racemates, enantiomers or diastereomers thereof, optionally in the form of the pharmacologically acceptable acid addition salts, the solvates and/or hydrates thereof.

Examples of ergot alkaloid derivatives are dihydroergotamine and ergotamine.

### List of reference numerals

- 1: inhaler
- 2: medicament preparation
- 3: container
- 4: bag
- 5: pressure generator
- 6: holder
- 7: drive spring
- 8: locking element
- 9: conveying tube
- 10: non-return valve
- 11: pressure chamber
- 12: delivery nozzle
- 13: mouthpiece
- 14: aerosol
- 15: supply air opening
- 16: first housing part (upper part)
- 17: inner part
- 17a: upper part of the inner part
- 17b: lower part of the inner part
- 18: second housing part (lower part)
- 19: retaining element
- 20: spring (in the lower housing part)
- 21: container base
- 22: piercing element
- 23: add-on device
- 24: chamber
- 25: housing
- 26: connecting member
- 27: delivery member
- 28: valve
- 29: add-on mouthpiece
- 30: tube
- 31: face mask
- 32: valve

## Claims

1. Portable inhaler (1) for the propellant-free nebulisation of a medicament preparation (2), having a pressure generator (5) for conveying and nebulising the medicament preparation (2), wherein the pressure generator is constructed as a pump and/or operating mechanically, the inhaler (1) further comprising a mouthpiece (13) having at least one supply air opening (15), and a delivery nozzle (12) for delivering the nebulised medicament preparation (2) as an aerosol (14) into the mouthpiece (13), **characterised in that** the inhaler (1) has an add-on device (23) with a chamber (24) for intermediate storage of the aerosol (14), wherein the chamber (24) is arranged or adapted to be arranged downstream of the delivery nozzle (12), wherein the chamber (24) is at least substantially cylindrical, elongate or conical in construction, wherein the add-on device (23) or an housing (25) thereof has a connecting member (26) for connecting to the mouthpiece (13), and wherein the at least one supply air opening (15) remains open when the add-on device (23) is attached to the mouthpiece (13).

2. Inhaler according to claim 1, **characterised in that** the chamber (24) has a larger cross section than the mouthpiece (13) and/or widens out at least in parts towards the delivery end of the add-on device (23).

3. Inhaler according to claim 1 or 2, **characterised in that** the add-on device (23) can be fitted onto the mouthpiece (13) preferably by clamping and/or is removable from the inhaler (1) or mouthpiece (13).

4. Inhaler according to one of the preceding claims, **characterised in that** the chamber (24) has a volume of more than at least 0.11, preferably more than 0.2 1, more particularly of about 0.2 to 0.6 1.

5. Inhaler according to one of the preceding claims, **characterised in that** the mouthpiece (13) has two supply air openings (15) that are arranged below the delivery nozzle (12) regarding their position along the longitudinal axis of the inhaler (1) whereas the main part of the chamber (24) for intermediate storage of the aerosol (14) extends above the delivery nozzle (12).

6. Inhaler according to one of the preceding claims, **characterised in that** the add-on device (23) comprises in particular at the delivery end a valve (28) for preventing air from flowing back into the chamber (24) and/or the mouthpiece (13) and/or for sucking in the aerosol and/or at least one valve (32) for blowing out exhaled air.

7. Inhaler according to one of the preceding claims, **characterised in that** the add-on device (23) is equipped or adapted to be equipped preferably selectively with an add-on mouthpiece (29), a tube (30) and/or a face mask (31).

8. Inhaler according to one of the preceding claims, **characterised in that** the inhaler (1) is a soft mist inhaler (SMI) which is constructed so that the aerosol (14) is delivered at a speed of less than 2 m/s, preferably of about 1.6 m/s or less, at a spacing of 10 cm from the delivery nozzle (12).

9. Inhaler according to claim 4, **characterised in that** the inhaler (1) is constructed so that it delivers and nebulises 10 to 50 µl of the medicament preparation (2) over a period of at least 1 s, particularly over 1.5 s, on each actuation or as the dose.

10. Inhaler according to one of the preceding claims, **characterised in that** the inhaler (1) is constructed so as to nebulise defined amounts of the medicament preparation (2) under pressures of 10 to 60 MPa.

11. Inhaler according to one of the preceding claims, **characterised in that** the pressure generation or nebulisation is carried out by spring force.

## Patentansprüche

1. Tragbarer Inhalator (1) zur treibgasfreien Zerstäubung einer Arzneimittelzubereitung (2), mit einem Druckerzeuger (5) zur Förderung und Zerstäubung der Arzneimittelzubereitung (2),
wobei der Druckerzeuger (5) als Pumpe ausgebildet ist und/oder mechanisch arbeitet,
wobei der Inhalator (1) des Weiteren ein Mundstück mit mindestens einer Zuluftöffnung (15) und eine Austragsdüse (12) zur Ausgabe der zerstäubten Arzneimittelzubereitung (2) als Aerosol (14) in das Mundstück (13) aufweist,
**dadurch gekennzeichnet,**
**dass** der Inhalator (1) eine Zusatzeinrichtung (23) mit einer Kammer (24) zur Zwischenspeicherung des Aerosols (14) hat, wobei die Kammer (24) stromab der Austragsdüse (12) angeordnet oder anordenbar ist, wobei die Kammer (24) zumindest im Wesentlichen zylindrisch, länglich oder konisch ausgebildet ist,
wobei die Zusatzeinrichtung (23) oder ein Gehäuse (25) derselben ein Anschlussstück (26) zur Verbindung mit dem Mundstück (13) hat, und
wobei die mindestens eine Zuluftöffnung (15) offen bleibt, wenn die Zusatzeinrichtung (23) am Mundstück (13) angebracht ist.

2. Inhalator nach Anspruch 1, **dadurch gekennzeichnet, dass** die Kammer (24) gegenüber dem Mundstück (13) im Querschnitt vergrößert ist und/oder sich zumindest abschnittsweise zum Abgabeende der Zusatzeinrichtung (23) hin erweitert.

3. Inhalator nach Anspruch 1 oder 2, **dadurch gekennzeichnet, dass** die Zusatzeinrichtung (23) auf das Mundstück (13) vorzugsweise klemmend aufsteckbar und/oder von dem Inhalator (1) oder Mundstück (13) lösbar ist.

4. Inhalator nach einem der voranstehenden Ansprüche, **dadurch gekennzeichnet, dass** die Kammer (24) ein Volumen von mehr als mindestens 0,1 1, vorzugsweise mehr als 0,2 1, insbesondere von etwa 0,2 bis 0,6 1, aufweist.

5. Inhalator nach einem der voranstehenden Ansprüche, **dadurch gekennzeichnet, dass** das Mundstück (13) zwei Zuluftöffnung (15) aufweist, die in Bezug auf ihre Position entlang der Längsachse des Inhalators unterhalb der Austragsdüse (12) angeordnet sind, wohingegen sich der Hauptteil der Kammer (24) zur Zwischenspeicherung des Aerosols (14) oberhalb der Austragsdüse (12) erstreckt.

6. Inhalator nach einem der voranstehenden Ansprüche, **dadurch gekennzeichnet, dass** die Zusatzeinrichtung (23) insbesondere abgabeseitig ein Ventil (28) zum Verhindern eines Rückströmens von Luft in die Kammer (24) und/oder das Mundstück (13) und/oder zum Absaugen des Aerosols und/oder mindestens ein Ventil (32) zum Ausblasen von Ausatemluft aufweist.

7. Inhalator nach einem der voranstehenden Ansprüche, **dadurch gekennzeichnet, dass** die Zusatzeinrichtung (23) vorzugsweise wahlweise mit einem Zusatzmundstück (29), einem Tubus (30) und/oder einer Gesichtsmaske (31) ausgerüstet oder ausrüstbar ist.

8. Inhalator nach einem der voranstehenden Ansprüche, **dadurch gekennzeichnet, dass** der Inhalator (1) ein Soft-Mist-Inhalator (SMI) ist, der derart ausgebildet ist, dass das Aerosol (14) mit einer Geschwindigkeit von weniger als 2 m/s in einem Abstand von 10 cm von der Austragsdüse (12) ausgegeben wird.

9. Inhalator nach einem Anspruch 4, **dadurch gekennzeichnet, dass** der Inhalator (1) derart ausgebildet ist, dass er pro Betätigung oder als Dosis jeweils 10 bis 50 µl der Arzneimittelzubereitung (2) über eine Zeitdauer von mindestens 1 s, insbesondere über 1,5 s, ausgibt und zerstäubt.

10. Inhalator nach einem der voranstehenden Ansprüche, **dadurch gekennzeichnet, dass** der Inhalator (1) derart ausgebildet ist, dass er definierte Mengen der Arzneimittelzubereitung (2) unter Drücken von 10 bis 60 MPa zerstäubt.

11. Inhalator nach einem der voranstehenden Ansprüche, **dadurch gekennzeichnet, dass** die Druckerzeugung bzw. Zerstäubung durch Federkraft erfolgt.

## Revendications

1. Inhalateur (1) portable pour la nébulisation sans gaz propulseur d'une préparation médicamenteuse (2), ayant un générateur de pression (5) pour transporter et nébuliser la préparation médicamenteuse (2), dans lequel le générateur de pression est conçu comme une pompe et/ou fonctionne mécaniquement,
l'inhalateur (1) comprenant en outre une pièce buccale (13) ayant au moins une ouverture d'alimentation en air (15), et
une buse de distribution (12) pour distribuer la préparation médicamenteuse (2) nébulisée comme un aérosol (14) dans la pièce buccale (13),
**caractérisé en ce que**
l'inhalateur (1) a un dispositif accessoire (23) avec une chambre (24) pour un stockage intermédiaire de l'aérosol (14), dans lequel la chambre (24) est agencée ou adaptée pour être agencée en aval de la buse de distribution (12), dans lequel la chambre (24) est de conception au moins sensiblement cylindrique, allongée ou conique,
dans lequel le dispositif accessoire (23) ou un boîtier (25) de celui-ci a un élément de connexion (26) pour se connecter à la pièce buccale (13), et dans lequel
l'au moins une ouverture d'alimentation en air (15) reste ouverte lorsque le dispositif accessoire (23) est fixé à la pièce buccale (13).

2. Inhalateur selon la revendication 1, **caractérisé en ce que** la chambre (24) a une section transversale plus grande que la pièce buccale (13) et/ou s'élargit au moins en partie vers l'extrémité de distribution du dispositif accessoire (23).

3. Inhalateur selon la revendication 1 ou 2, **caractérisé en ce que** le dispositif accessoire (23) peut être monté sur la pièce buccale (13) de préférence par serrage et/ou est amovible de l'inhalateur (1) ou de la pièce buccale (13).

4. Inhalateur selon l'une quelconque des revendications précédentes, **caractérisé en ce que** la chambre (24) a un volume supérieur à au moins 0,11, de préférence supérieur à 0,21, plus particulièrement d'environ 0,2 à 0,61.

5. Inhalateur selon l'une quelconque des revendications précédentes, **caractérisé en ce que** la pièce buccale (13) a deux ouvertures d'alimentation en air (15) qui sont agencées sous la buse de distribution (12) concernant leur position le long de l'axe longitudinal de l'inhalateur (1) alors que la partie principale de la chambre (24) pour un stockage intermédiaire de l'aérosol (14) s'étend au-dessus de la buse de distribution (12).

6. Inhalateur selon l'une quelconque des revendications précédentes, **caractérisé en ce que** le dispositif accessoire (23) comprend en particulier à l'extrémité de distribution une soupape (28) pour empêcher l'air de refluer dans la chambre (24) et/ou la pièce buccale (13) et/ou pour créer une aspiration dans l'aérosol et/ou au moins une soupape (32) pour souffler l'air expiré.

7. Inhalateur selon l'une quelconque des revendications précédentes, **caractérisé en ce que** le dispositif accessoire (23) est équipé ou adapté pour être équipé de préférence sélectivement d'une pièce buccale accessoire (29), d'un tube (30) et/ou d'un masque facial (31).

8. Inhalateur selon l'une quelconque des revendications précédentes, **caractérisé en ce que** l'inhalateur (1) est un inhalateur à brouillard doux (SMI) qui est conçu de sorte que l'aérosol (14) soit distribué à une vitesse inférieure à 2 m/s, de préférence d'environ 1,6 m/s ou moins, à un espacement de 10 cm de la buse de distribution (12).

9. Inhalateur selon la revendication 4, **caractérisé en ce que** l'inhalateur (1) est conçu de sorte qu'il distribue et nébulise 10 à 50 µl de la préparation médicamenteuse (2) pendant une période d'au moins 1 s, en particulier pendant 1,5 s, à chaque actionnement ou comme dose.

10. Inhalateur selon l'une quelconque des revendications précédentes, **caractérisé en ce que** l'inhalateur (1) est conçu de sorte à nébuliser des quantités définies de la préparation médicamenteuse (2) sous des pressions de 10 à 60 MPa.

11. Inhalateur selon l'une quelconque des revendications précédentes, **caractérisé en ce que** la génération de pression ou la nébulisation s'effectue par force élastique.
